# EUROPEAN PATENT APPLICATION

(11) **EP 4 163 376 A1**
(43) Date of publication of application: **12.04.2023**
(21) Application number: 21821284.3
(22) Date of filing: 09.06.2021
(51) Int. Cl.: C12N 15/17, C12P 21/02, C07K 14/62, C07K 19/00, C12N 15/70

(54) **INSULIN ASPART DERIVATIVE, AND PREPARATION METHOD THEREFOR AND USE THEREOF**

(30) Priority: 09.06.2020 CN 202010526819
(71) Applicant: Ningbo Kunpeng Biotech Co., Ltd., Yuyao Ningbo, Zhejiang 315400 (CN)
(72) Inventor: LIU, Huiling, Ningbo, Zhejiang 315400 (CN); LUO, Li, Ningbo, Zhejiang 315400 (CN); TANG, Yalian, Ningbo, Zhejiang 315400 (CN)
(74) Representative: Lavoix
(86) International application number: PCT/CN2021/099244
(87) International publication number: WO 2021/249444

(57) **Abstract**

An insulin aspart derivative and a preparation method therefor. The derivative comprises a fusion protein of a green fluorescent protein folding unit and an insulin aspart precursor or an active fragment thereof. The expression level of the fusion protein is significantly increased, and the insulin aspart precursor protein in the fusion protein is folded correctly and has a biological activity. In addition, the green fluorescent protein folding unit in the fusion protein can be digested into small fragments by a protease, which has a large molecular weight difference compared to the target protein and is thus easy to separate. Further provided is a method for preparing the insulin aspart and an intermediate by means of using the fusion protein.

## Description

### TECHNICAL FIELD

The present invention relates to the field of biotechnology, in particular to an insulin aspart derivative and use thereof.

### BACKGROUND

Diabetes is a major disease that threatens human health worldwide. In China, with the change of people's lifestyle and the acceleration of aging, the prevalence of diabetes is increasing rapidly. Acute and chronic complications of diabetes, especially the chronic complications, involve multiple organs, cause high disability and mortality rates, seriously affect the physical and mental health of patients, and bring heavy burdens to individuals, families and society.

As a rapid-acting insulin analogue, insulin aspart belongs to the third generation of insulin, which is formed with two chains A and B bridged by two hemiamino acid disulfide bonds, wherein genetic engineering DNA recombinant technology is used to replace (mutate) the proline (Pro) at position 28 of the human insulin B chain with negative charged aspartic acid (Asp) to prevent the self-polymerization of insulin monomers or dimers (formed by B28 of one insulin molecule and B23 of another insulin molecule) through the repulsion of charges, so that the polymerization between molecules is reduced. It can simulate the secretion mode of human insulin well and its pharmacokinetic characteristics are about half of the conventional human insulin. Its onset time is 10-20 minutes, peak time is 40 minutes, and the duration of function is shortened to 3-5 hours, so that patients can obtain good blood glucose control while barely superimpose with insulin before meals or at night, which significantly reduces the incidence of nocturnal hypoglycemia.

The preparation of insulin aspart generally adopts genetic engineering technology to prepare precursors. The first-researching company Novo Nordisk uses Saccharomyces cerevisiae as the expression host to produce insulin aspart precursors by using recombinant DNA technology, and then prepares insulin aspart through a series of complex processes such as transpeptide. Such method is technically difficult and complex in the aspect of host bacteria transformation. Due to the weak expression ability of Saccharomyces cerevisiae, the yield of insulin aspart is not high. The maximum shaking culture of engineered bacteria is 21.5mg/L, which partly increases the cost of drug production.

Therefore, there is an urgent need in this field to develop a preparation and purification method of insulin aspart with simple process, environmental friendliness and high yield.

### SUMMARY OF THE INVENTION

The purpose of the present invention is to provide an insulin aspart derivative and use thereof.

In the first aspect of the present invention, it provides an insulin aspart fusion protein having the structure as shown in Formula I:

A-FP-TEV-R-G (I)

wherein,
"-" represents a peptide bond;
A is absent or a leader peptide,
FP is a green fluorescent protein folding unit,
TEV is a restriction site, and preferably is a restriction site of TEV enzyme;
R is arginine or lysine used for enzyme digestion;
G is a precursor of insulin aspart or an active fragment thereof;
wherein the green fluorescent protein folding unit comprises 2-6, preferably 2-3 β-folding unit selected from the group consisting of:

| β-folding unit | Amino acid sequence |
|---|---|
| u1 | VPILVELDGDVNG (SEQ ID NO: 11) |
| u2 | HKFSVRGEGEGDAT (SEQ ID NO: 12) |
| u3 | KLTLKFICTT (SEQ ID NO: 13) |
| u4 | YVQERTISFKD (SEQ ID NO: 14) |
| u5 | TYKTRAEVKFEGD (SEQ ID NO: 15) |
| u6 | TLVNRIELKGIDF (SEQ ID NO: 16) |
| u7 | HNVYITADKQ (SEQ ID NO: 17) |
| u8 | GIKANFKIRHNVED (SEQ ID NO: 18) |
| u9 | VQLADHYQQNTPIG (SEQ ID NO: 19) |
| u10 | HYLSTQSVLSKD (SEQ ID NO: 20) |
| u11 | HMVLLEFVTAAGI (SEQ ID NO: 21). |

In another preferred embodiment, the green fluorescent protein folding unit is u8-u9, u9-u10-u11, or u10-u11.

In another preferred embodiment, the G is a Boc-modified insulin aspart precursor having the structure as shown in Formula II:

GB-X-GA (II)

wherein,
GB is the Boc-modified B chain of insulin aspart whose amino acid sequence is shown in positions 1-30 of SEQ ID NO: 5,
X is a linker peptide, and preferably the amino acid sequence of X is R, RR, RRR or as shown in SEQ ID NO: 6-9 (RRGSKR, RRAAKR, RRYPGDVKR or RREAEDLQVGQVELGGGPGAGSLQPLALEGSLQKR).
GA is the A chain of insulin aspart whose amino acid sequence is shown in positions 32-52 of SEQ ID NO: 5.

In another preferred embodiment, the R is used for trypsin digestion and carboxypeptidase digestion.

In another preferred embodiment, the G is a Boc-modified insulin aspart whose sequence is shown in SEQ ID NO: 5.

In another preferred embodiment, an intrachain disulfide bond exists between GB-X-GA.

In another preferred embodiment, the sequence of the insulin aspart fusion protein is shown in SEQ ID NOs: 1, 22, 23.

In another preferred embodiment, interchain disulfide bonds are formed between position 7 of B chain and position 7 of A chain (A7-B7), and between position 19 of B chain and position 20 of A chain (A20-B19) in the insulin aspart.

In another preferred embodiment, an intrachain disulfide bond is formed between position 6 of A chain and position 11 of A chain (A6-A11) in the insulin aspart.

In the second aspect of the present invention, it provides a double chain insulin aspart fusion protein having the structure as shown in Formula III:

A-FP-TEV-R-D (III)

wherein,
"-" represents a peptide bond;
A is absent or a leader peptide, preferably is a leader peptide whose sequence is shown in SEQ ID NO: 2,
FP is a green fluorescent protein folding unit,
TEV is a restriction site, and preferably is a restriction site of TEV enzyme (whose sequence is ENLYFQG, SEQ ID NO: 4);
R is arginine or lysine used for enzyme digestion;
D is a Boc-modified double chain insulin aspart having the structure as shown in the following Formula IV; 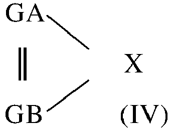
wherein, " 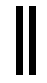 " represents a disulfide bond;
GA is the A chain of insulin aspart whose amino acid sequence is shown in positions 32-52 of SEQ ID NO: 5,
X is a linker peptide;
GB is the B chain of insulin aspart modified with Boc at position 29, whose amino acid sequence is shown in positions 1-30 of SEQ ID NO: 5;
wherein the green fluorescent protein folding unit comprises 2-6, preferably 2-3 β-folding unit selected from the group consisting of:

| β-folding unit | Amino acid sequence |
|---|---|
| u1 | VPILVELDGDVNG (SEQ ID NO: 11) |
| u2 | HKFSVRGEGEGDAT (SEQ ID NO: 12) |
| u3 | KLTLKFICTT (SEQ ID NO: 13) |
| u4 | YVQERTISFKD (SEQ ID NO: 14) |
| u5 | TYKTRAEVKFEGD (SEQ ID NO: 15) |
| u6 | TLVNRIELKGIDF (SEQ ID NO: 16) |
| u7 | HNVYITADKQ (SEQ ID NO: 17) |
| u8 | GIKANFKIRHNVED (SEQ ID NO: 18) |
| u9 | VQLADHYQQNTPIG (SEQ ID NO: 19) |
| u10 | HYLSTQSVLSKD (SEQ ID NO: 20) |
| u11 | HMVLLEFVTAAGI (SEQ ID NO: 21). |

In another preferred embodiment, the green fluorescent protein folding unit is u8-u9, u9-u10-u11, or u10-u11.

In another preferred embodiment, the amino acid sequence of the green fluorescent protein folding unit is shown in SEQ ID NOs: 3, 24, 25.

In another preferred embodiment, the C-terminus of the B chain of insulin aspart is connected to the N-terminus of the A chain of insulin aspart through a linker peptide.

In another preferred embodiment, the X is a linker peptide, and preferably the amino acid sequence of X is R, RR, RRR or as shown in SEQ ID NO: 6-9 (RRGSKR, RRAAKR, RRYPGDVKR or RREAEDLQVGQVELGGGPGAGSLQPLALEGSLQKR).

In the third aspect of the present invention, it provides a Boc-modified insulin aspart precursor having the structure as shown in Formula II:

GB- X-GA (II)

wherein,
GB is the B chain of insulin aspart modified with Boc at position 29, whose amino acid sequence is shown in positions 1-30 of SEQ ID NO: 5,
X is a linker peptide, and preferably the amino acid sequence of X is R, RR, RRR or as shown in SEQ ID NO: 6-9 (RRGSKR, RRAAKR, RRYPGDVKR or RREAEDLQVGQVELGGGPGAGSLQPLALEGSLQKR).
GA is the A chain of insulin aspart whose amino acid sequence is shown in positions 32-52 of SEQ ID NO: 5.

In another preferred embodiment, the protected lysine is a Nε-(tert-butoxycarbonyl)-lysine.

In the fourth aspect of the present invention, it provides a Boc-modified insulin aspart having the structure as shown in Formula IV: 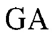 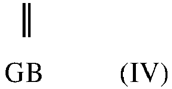 wherein, " 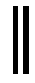 " represents a disulfide bond;
GA is the A chain of insulin aspart whose amino acid sequence is shown in positions 32-52 of SEQ ID NO: 5,
GB is the B chain of insulin aspart whose amino acid sequence is shown in positions 1-30 of SEQ ID NO: 5, and the lysine at position 29 of the B chain is Nε-(tert-butoxycarbonyl)-lysine.

In the fifth aspect of the present invention, it provides a method for preparing insulin aspart, comprising the steps:
(i) using recombinant bacteria to ferment to prepare insulin aspart fusion protein (primary protein) fermentation broth;
(ii) digesting the insulin aspart fusion protein (primary protein) with enzyme, thereby obtaining a Mixture I containing Boc-modified insulin aspart (secondary protein);
(iii) deprotecting the Boc-modified insulin aspart (secondary protein), thereby obtaining a Mixture II containing deprotected insulin aspart (tertiary protein);
(iv) purifying the Mixture II, thereby obtaining the insulin aspart.

In another preferred embodiment, between the step (ii) and step (iii) it further comprises the step:
(I) performing the first anion exchange chromatography on the Mixture I, thereby obtaining dry powder containing the Boc-modified insulin aspart (secondary protein).

In another preferred embodiment, the purification in step (iv) further comprises the steps:
(II) performing the second anion exchange chromatography using glycine as the mobile phase on the Mixture II, thereby obtaining an Eluate II containing the deprotected insulin aspart (tertiary protein).
(III) performing reversed-phase chromatography on the Eluate II, thereby obtaining the insulin aspart.

In another preferred embodiment, the purity of the produced insulin aspart is over 99%.

In another preferred embodiment, the produced insulin aspart has insulin aspart bioactivity.

In another preferred embodiment, the Boc-insulin aspart is insulin aspart with a protected lysine on B29 (position 29 of the insulin B chain).

In another preferred embodiment, the protected lysine is a lysine containing a protective group.

In another preferred embodiment, the protected lysine is a Nε-(tert-butoxycarbonyl)-lysine.

In another preferred embodiment, in step (i), recombinant bacteria are used for fermentation and production of insulin aspart fusion protein.

In another preferred embodiment, the recombinant bacteria comprise or are integrated with an expression cassette expressing the insulin aspart fusion protein.

In another preferred embodiment, in step (i), insulin aspart fusion protein inclusion bodies are isolated from the fermentation broth of the recombinant bacteria.

In another preferred embodiment, in step (i), it further comprises a step of denaturing and renaturing the inclusion bodies, thereby obtaining the insulin aspart fusion protein (primary protein) with correct protein folding.

In another preferred embodiment, in the insulin aspart fusion protein with correct protein folding, an intrachain disulfide bond is comprised between A and B chains of the insulin aspart.

In another preferred embodiment, the insulin aspart fusion protein is as described in the first aspect of the present invention.

In another preferred embodiment, in step (ii), trypsin and carboxypeptidase B are used for the digestion.

In another preferred embodiment, in step (ii), the mass ratio of carboxypeptidase B to insulin aspart fusion protein is 1:(20000-30000).

In another preferred embodiment, in step (ii), the trypsin is a recombinant porcine trypsin.

In another preferred embodiment, in step (ii), the mass ratio of trypsin to insulin aspart fusion protein is 1:1000-10000, preferably 1:1000-3000.

In another preferred embodiment, in step (ii), the temperature for digestion is 32-42°C preferably 36-38°C.

In another preferred embodiment, in step (ii), the time for digestion is 10-30 h, preferably 14-20 h.

In another preferred embodiment, in step (ii), the pH of the insulin aspart fusion protein digestion system is 7.0-9.5, preferably 8.0-9.5.

In another preferred embodiment, in step (iii), TFA (trifluoroacetic acid) is added to the reaction system for the deprotection.

In another preferred embodiment, in step (iii), the ratio of Boc-modified insulin aspart (secondary protein) to TFA is 1g : (4-10mL).

In another preferred embodiment, in step (iii), the temperature for deprotection reaction is 4-37°C, preferably 18-25°C.

In another preferred embodiment, in step (iii), the time for deprotection reaction is 0.5-8 h, preferably 0.5-3 h.

In another preferred embodiment, the Boc-insulin aspart is a Nε-(tert-butoxycarbonyl)-lysine insulin aspart.

In another preferred embodiment, in step (I), the loading amount of Boc-modified insulin aspart (secondary protein) is ≤ 45 mg/ml.

In another preferred embodiment, in step (I), 100-500 mmol/L of sodium chloride is used for linear gradient elution.

In another preferred embodiment, in step (II), 100-500 mmol/L of sodium chloride is used for isocratic and gradient elution.

In another preferred embodiment, in step (II), the loading amount of insulin aspart in Eluate I is ≤ 15mg/ml, and preferably the loading amount is ≤10mg/ml.

In another preferred embodiment, in step (III), 150-250 mmol/L, preferably 180-220 mmol/L acetonitrile solution of sodium acetate is used as mobile phase for isocratic elution.

In another preferred embodiment, in step (III), the loading amount of insulin aspart in Eluate II is ≤ 6 mg/ml, and preferably the loading amount is ≤ 5 mg/ml.

In another preferred embodiment, after step (III), it further comprises a step of crystallization and lyophilization of the prepared insulin aspart, to prepare a lyophilized product.

In the sixth aspect of the present invention, it provides an insulin aspart formulation prepared by using the method of the fifth aspect of the present invention.

In another preferred embodiment, the purity of the insulin aspart contained in the insulin aspart formulation is over 99%.

In another preferred embodiment, the insulin aspart contained in the insulin aspart formulation has bioactivity.

In the seventh aspect of the present invention, it provides an isolated polynucleotide encoding the insulin aspart fusion protein of the first aspect of the present invention, the insulin aspart main chain fusion protein of the second aspect of the present invention, the Boc-modified insulin aspart precursor of the third aspect of the present invention, or the Boc-modified insulin aspart main chain of the fourth aspect of the present invention.

In the eighth aspect of the present invention, it provides a vector comprising the polynucleotide of the seventh aspect of the present invention.

In another preferred embodiment, the vector is selected from the group consisting of DNA, RNA, a plasmid, a lentiviral vector, an adenoviral vector, a retroviral vector, a transposon, and a combination thereof.

In the ninth aspect of the present invention, it provides a host cell comprising the vector of the eighth aspect of the present invention, or in which the chromosome is integrated with exogenous polynucleotide of the seventh aspect of the present invention, or expressing the insulin aspart fusion protein of the first aspect of the present invention, the insulin aspart main chain fusion protein of the second aspect of the present invention, the Boc-modified insulin aspart precursor of the third aspect of the present invention, or the Boc-modified insulin aspart main chain of the fourth aspect of the present invention.

In another preferred embodiment, the host cell is *Escherichia coli, Bacillus subtilis,* a yeast cell, an insect cell, a mammalian cell and a combination thereof.

In the tenth aspect of the present invention, it provides a formulation or pharmaceutical composition comprising the insulin aspart fusion protein of the first aspect of the present invention, the insulin aspart main chain fusion protein of the second aspect of the present invention, the Boc-modified insulin aspart precursor of the third aspect of the present invention, or the Boc-modified insulin aspart main chain of the fourth aspect of the present invention, and a pharmaceutically acceptable carrier.

It should be understood that, within the scope of the present invention, the technical features aforementioned and specifically described below (such as in the examples) can be combined with each other, thereby constituting new or preferred technical solutions which are not described here one by one due to space constraints.

### DESCRIPTION OF THE FIGURES

Figure 1 shows a map of the plasmid pBAD-FP-TEV-R-G.
Figure 2 shows a map of the plasmid pEvol-pylRs-pylT.
Figure 3 shows the SDS-PAGE electropherogram of the insulin aspart fusion protein after denaturation and renaturation of the inclusion bodies.
Figure 4 shows the SDS-PAGE electropherogram of the Boc-insulin aspart after the first chromatography.
Figure 5 shows the HPLC detection spectrogram of the insulin aspart after deprotection.
Figure 6 shows the HPLC detection spectrogram of the insulin aspart after the second chromatography.
Figure 7 shows the HPLC detection spectrogram of the insulin aspart after the third chromatography.
Figure 8 shows the crystal diagram of the insulin aspart crystal.
Figure 9 shows the mass spectrum of the Boc-insulin aspart.

### DETAILED DESCRIPTION

After extensive and intensive research, the inventors have discovered an insulin aspart derivative and preparation method therefor. Specifically, the present invention provides a fusion protein comprising a green fluorescent protein folding unit and an insulin aspart precursor or an active fragment thereof. The fusion protein of the present invention has a significantly increased expression level, and the insulin aspart protein in the fusion protein is folded correctly, and has biological activity. Moreover, the green fluorescent protein folding unit in the fusion protein of the present invention can be digested into small fragments by a protease, which have a great difference in molecular weight in comparison to the target protein, and are easy to separate. The present invention further provides a method for using the fusion protein to prepare insulin aspart and intermediates.

### Terms

In order to make it easier to understand the present disclosure, certain terms are firstly defined. As used in this application, unless expressly stated otherwise herein, each of the following terms shall have the meaning given below. Other definitions are stated throughout the application.

The term "about" may refer to a value or composition within an acceptable error range of a particular value or composition determined by a person having ordinary skill in the art, which will depend in part on how the value or composition is measured or determined.

### Insulin aspart

Insulin products are the first major drugs in the diabetes market, accounting for about 53% of the market share, of which the third generation of recombinant insulin is the mainstay. Insulin aspart belongs to the third generation of recombinant insulin and is a rapid-acting insulin (or mealtime insulin). After subcutaneous injection, it takes effect in 10-15 minutes, peaks in 1-2 hours, and lasts for 4-6 hours.

### Construction of the plasmid expressing insulin aspart

FP-TEV-R-G target gene was synthesized, which has recognition sites for restriction endonucleases Nco I and Xho I at both ends and contains gene A encoding insulin aspart. The codon of this sequence was optimized and can achieve high level expression of functional protein in *E. coli.* After expression, the restriction endonucleases Nco I and Xho I were used to cut the expression vector "pBAD/His A(Kana^{R})" and the plasmid containing the target gene "FP-TEV-R-G". The digested products were separated by agarose electrophoresis, and then extracted by agarose gel DNA recovery kit. Finally, the two DNA fragments were connected by T4 DNA ligase. The connected product was chemically transformed into *E. coli* Top10 cells, and the transformed cells were cultured in LB agar medium (10 g/L yeast peptone, 5 g/L yeast extract, 10 g/L NaCl, 1.5% agar) containing 50 µg/mL kanamycin overnight. Three live colonies were picked and cultured in 5mL liquid LB medium (10g/L yeast peptone, 5g/L yeast extract and 10g/L NaCl) containing 50µg/mL kanamycin overnight, and the plasmid was extracted by using small amount plasmid extraction kit. Then, the extracted plasmid was sequenced to confirm correct insertion. The finally obtained plasmid was named as "pBAD-FP-TEV-R-G".

### Fusion protein

By using the green fluorescent protein folding unit, the invention constructs two fusion proteins, namely the insulin aspart fusion protein containing single chain insulin aspart according to the first aspect of the present invention and the double chain insulin aspart fusion protein containing double chain insulin aspart according to the second aspect of the present invention. In fact, the protection scope of the two fusion proteins of the present invention may overlap partially. For example, the C-terminus of the B chain of the double chain insulin aspart contained in the fusion protein can be connected to the N-terminus of the A chain through a linker peptide, which can also be considered as a single chain containing an intrachain disulfide bond.

The green fluorescent protein folding unit contained in the fusion protein of the present invention comprises 2-6, preferably 2-3 β-folding units selected from the group consisting of:

| | Amino acid sequence |
|---|---|
| u1 | VPILVELDGDVNG (SEQ ID NO: 11) |
| u2 | HKFSVRGEGEGDAT (SEQ ID NO: 12) |
| u3 | KLTLKFICTT (SEQ ID NO: 13) |
| u4 | YVQERTISFKD (SEQ ID NO: 14) |
| u5 | TYKTRAEVKFEGD (SEQ ID NO: 15) |
| u6 | TLVNRIELKGIDF (SEQ ID NO: 16) |
| u7 | HNVYITADKQ (SEQ ID NO: 17) |
| u8 | GIKANFKIRHNVED (SEQ ID NO: 18) |
| u9 | VQLADHYQQNTPIG (SEQ ID NO: 19) |
| u10 | HYLSTQSVLSKD (SEQ ID NO: 20) |
| u11 | HMVLLEFVTAAGI (SEQ ID NO: 21). |

In another preferred embodiment, the green fluorescent protein folding unit FP may be selected from the group consisting of: u8, u9, u2-u3, u4-u5, u8-u9, u1-u2-u3, u2-u3-u4, u3-u4-u5, u5-u6-u7, u8-u9-u10, u9-u10-u11, u3-u5-u7, u3-u4-u6, u4-u7-u10, u6-u8-u10, u1-u2-u3-u4, u2-u3-u4-u5, u3-u4-u3-u4, u3-u5-u7-u9, u5-u6-u7-u8, u1-u3-u7-u9, u2-u2-u7-u8, u7-u2-u5-u11, u3-u4-u7-u10, u1-I-u2, u1-I-u5, u2-I-u4, u3-I-u8, u5-I-u6, or u10-I-u11.

In another preferred embodiment, the green fluorescent protein folding unit is u8-u9, u9-u10-u11, or u10-u11.

As used herein, the term "fusion protein" also includes variant forms having the above-mentioned activities. These variant forms include (but are not limited to): 1-3 (usually 1-2, more preferably 1) amino acid deletions, insertions and/or substitutions, and one or several (usually 3 or less, preferably 2 or less, more preferably 1 or less) amino acids added or deleted at the C-terminal and/or N-terminal. For example, in this field, when substituted with amino acids with close or similar properties, the function of the protein is usually not changed. For another example, adding or deleting one or several amino acids at the C-terminus and/or N-terminus usually does not change the structure and function of the protein. In addition, the term also includes the polypeptide of the present invention in monomeric and multimeric forms. The term also includes linear and non-linear polypeptides (such as cyclic peptides).

The present invention also includes active fragments, derivatives and analogs of the above-mentioned fusion protein. As used herein, the terms "fragment", "derivative" and "analog" refer to a polypeptide that substantially retains the function or activity of the fusion protein of the present invention. The polypeptide fragments, derivatives or analogs of the present invention can be (i) a polypeptide in which one or more conservative or non-conservative amino acid residues (preferably conservative amino acid residues) are substituted, or (ii) a polypeptide with a substitution group in one or more amino acid residues, or (iii) a polypeptide formed by fusion of a polypeptide with another compound (such as a compound that prolongs the half-life of polypeptide, such as polyethylene glycol), or (iv) the polypeptide formed by fusion of additional amino acid sequence to this polypeptide sequence (fusion protein formed by fusion with a tag sequence such as leader sequence, secretory sequence or 6His). According to the teachings herein, these fragments, derivatives and analogs fall within the scope well known to those skilled in the art.

A preferred type of active derivative means that compared with the amino acid sequence of the present invention, at most 3, preferably at most 2, and more preferably at most 1 amino acid are replaced by amino acids with close or similar properties to form a polypeptide. These conservative variant polypeptides are best produced according to Table A by performing amino acid substitutions.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the fusion protein of the present invention. The difference between these analogs and the polypeptide of the present invention may be a difference in amino acid sequence, may also be a difference in modified form that does not affect the sequence, or both. Analogs also include analogs having residues different from natural L-amino acids (such as D-amino acids), and analogs having non-naturally occurring or synthetic amino acids (such as β, γ-amino acids). It should be understood that the polypeptide of the present invention is not limited to the representative polypeptides exemplified above.

In addition, the fusion protein of the present invention can also be modified. Modification (usually without changing the primary structure) forms include: chemically derivative forms of polypeptides in vivo or in vitro, such as acetylation or carboxylation. Modifications also include glycosylation, such as those polypeptides produced by glycosylation modifications during the synthesis and processing of the polypeptide or during further processing steps. This modification can be accomplished by exposing the polypeptide to an enzyme that performs glycosylation (such as a mammalian glycosylase or deglycosylase). Modification forms also include sequences with phosphorylated amino acid residues (such as phosphotyrosine, phosphoserine, phosphothreonine). It also includes polypeptides that have been modified to improve their anti-proteolytic properties or optimize their solubility properties.

The term "polynucleotide encoding the fusion protein of the present invention" may include a polynucleotide encoding the fusion protein of the present invention, or a polynucleotide that also includes additional coding and/or non-coding sequences.

The present invention also relates to variants of the above-mentioned polynucleotides, which encode fragments, analogs and derivatives of polypeptides or fusion proteins having the same amino acid sequence as the present invention. These nucleotide variants include substitution variants, deletion variants and insertion variants. As known in the art, an allelic variant is an alternative form of polynucleotide. It may be a substitution, deletion or insertion of one or more nucleotides, but will not substantially change the function of the encoded fusion protein.

The present invention also relates to polynucleotides that hybridize with the aforementioned sequences and have at least 50%, preferably at least 70%, and more preferably at least 80% identity between the two sequences. The present invention particularly relates to polynucleotides that can hybridize with the polynucleotide of the present invention under strict conditions (or stringent conditions). In the present invention, "strict conditions" refer to: (1) hybridization and elution at lower ionic strength and higher temperature, such as 0.2×SSC, 0.1% SDS, 60°C; or (2) adding denaturant during hybridization, such as 50% (v/v) formamide, 0.1% calf serum/0.1% Ficoll, 42°C, etc.; or (3) hybridization occurs only when the identity between the two sequences is at least 90% or more, and more preferably 95% or more.

The fusion protein and polynucleotides of the present invention are preferably provided in an isolated form, and more preferably, are purified to homogeneity.

The full-length sequence of the polynucleotide of the present invention can usually be obtained by PCR amplification method, recombination method or artificial synthesis method. For the PCR amplification method, primers can be designed according to the relevant nucleotide sequence disclosed in the present invention, especially the open reading frame sequence, and a commercially available cDNA library or a cDNA library prepared according to a conventional method known to those skilled in the art is used as a template to amplify the relevant sequence. When the sequence is long, it is often necessary to perform two or more PCR amplifications, and then each amplified fragments are spliced together in the correct order.

Once the relevant sequences are obtained, the relevant sequences can be obtained in large quantities by recombination method. It is usually cloned into a vector, then transferred into a cell, and then the relevant sequence is isolated from the host cell after proliferation by conventional methods.

In addition, the relevant sequences can also be synthesized by artificial synthesis, especially when the fragment length is short. Usually, by first synthesizing multiple small fragments, and then ligating to obtain very long fragments.

At present, the DNA sequence encoding the protein (or fragment or derivative thereof) of the present invention can be obtained completely through chemical synthesis. The DNA sequence can then be introduced into various existing DNA molecules (or such as vectors) and cells known in the art.

The method of using PCR technology to amplify DNA/RNA is preferably used to obtain the polynucleotide of the present invention. Especially when it is difficult to obtain full-length cDNA from the library, the RACE method (RACE-cDNA end rapid amplification method) can be preferably used, and the primers used for PCR can be appropriately selected according to the sequence information of the present invention disclosed herein, and can be synthesized by conventional methods. The amplified DNA/RNA fragments can be separated and purified by conventional methods such as gel electrophoresis.

### Expression Vector

The present invention also relates to a vector containing the polynucleotide of the present invention, a host cell produced by genetic engineering using the vector of the present invention or the sequence encoding the fusion protein of the present invention, and a method for producing the polypeptide of the present invention through recombinant technology.

Through conventional recombinant DNA technology, the polynucleotide sequence of the present invention can be used to express or produce recombinant fusion protein. Generally, there are the following steps:
(1). using the polynucleotide (or variant) of the present invention encoding the fusion protein of the present invention, or using a recombinant expression vector containing the polynucleotide to transform or transduce a suitable host cell;
(2). culturing a host cell in a suitable medium;
(3). isolating and purifying protein from culture medium or cells.

In the present invention, the polynucleotide sequence encoding the fusion protein can be inserted into a recombinant expression vector. The term "recombinant expression vector" refers to bacterial plasmids, bacteriophages, yeast plasmids, plant cell viruses, mammalian cell viruses such as adenovirus, retrovirus or other vectors well known in the art. Any plasmid and vector can be used as long as it can be replicated and stabilized in the host. An important feature of an expression vector is that it usually contains an origin of replication, a promoter, a marker gene, and translation control elements.

Methods well known to those skilled in the art can be used to construct an expression vector containing the DNA sequence encoding the fusion protein of the present invention and appropriate transcription/translation control signals. These methods include *in vitro* recombinant DNA technology, DNA synthesis technology, and *in vivo* recombination technology. The DNA sequence can be effectively linked to an appropriate promoter in the expression vector to guide mRNA synthesis. Representative examples of these promoters are: *Escherichia coli* lac or trp promoter; lambda phage PL promoter; eukaryotic promoters including CMV immediate early promoter, HSV thymidine kinase promoter, early and late SV40 promoter, retroviral LTRs and some other known promoters that can control gene expression in prokaryotic or eukaryotic cells or viruses. The expression vector also includes a ribosome binding site for translation initiation and a transcription terminator.

In addition, the expression vector preferably contains one or more selectable marker genes to provide phenotypic traits for selecting transformed host cells, such as dihydrofolate reductase, neomycin resistance, and green fluorescent protein (GFP) for eukaryotic cell culture, or tetracycline or ampicillin resistance for *E. coli.*

A vector containing the above-mentioned appropriate DNA sequence and an appropriate promoter or control sequence can be used to transform an appropriate host cell so that it can express the protein.

The host cell can be a prokaryotic cell, such as a bacterial cell; or a lower eukaryotic cell, such as a yeast cell; or a higher eukaryotic cell, such as a mammalian cell. Representative examples include: *Escherichia coli*, *Streptomyces*; bacterial cells of *Salmonella typhimurium*; fungal cells such as yeast and plant cells (such as ginseng cells).

When the polynucleotide of the present invention is expressed in higher eukaryotic cells, if an enhancer sequence is inserted into the vector, the transcription will be enhanced. Enhancers are cis-acting factors of DNA, usually about 10 to 300 base pairs, acting on promoters to enhance gene transcription. Examples include the 100 to 270 base pair SV40 enhancer on the late side of the replication initiation point, the polyoma enhancer on the late side of the replication initiation point, and adenovirus enhancers and the like.

Those of ordinary skill in the art know how to select appropriate vectors, promoters, enhancers and host cells.

Transformation of host cells with recombinant DNA can be carried out by conventional techniques well known to those skilled in the art. When the host is a prokaryote such as *Escherichia coli*, competent cells that can absorb DNA can be harvested after the exponential growth phase and treated with the CaCl₂ method. The steps used are well known in the art. Another method is to use MgCl₂. If necessary, transformation can also be carried out by electroporation. When the host is a eukaryote, the following DNA transfection methods can be selected: calcium phosphate co-precipitation method, conventional mechanical methods such as microinjection, electroporation, liposome packaging, etc..

The obtained transformants can be cultured by conventional methods to express the polypeptide encoded by the gene of the present invention. Depending on the host cell used, the medium used in the culture can be selected from various conventional mediums. The culture is carried out under conditions suitable for the growth of the host cell. After the host cells have grown to an appropriate cell density, the selected promoter is induced by a suitable method (such as temperature conversion or chemical induction), and the cells are then cultured for a period of time.

The recombinant polypeptide in the above method can be expressed in the cell, on the cell membrane, or secreted out of the cell. If necessary, the physical, chemical, and other characteristics can be used to separate and purify the recombinant protein through various separation methods. These methods are well known to those skilled in the art. Examples of these methods include, but are not limited to: conventional renaturation treatment, treatment with protein precipitation agent (salting out method), centrifugation, bacteria broken through osmosis, ultra treatment, ultracentrifugation, molecular sieve chromatography (gel filtration), adsorption chromatography, ion exchange chromatography, high performance liquid chromatography (HPLC) and various other liquid chromatography techniques and combinations of these methods.

### The present invention mainly has the following advantages:

(1) The method of the present invention does not require dilution, ultrafiltration and other methods to remove the excess inorganic salts in the supernatant of the fermentation broth, and the purity of the obtained inclusion bodies is high, with relatively little pigment, which reduces the separation substance for subsequent purification and the purification cost. Moreover, the one-step yield of separating insulin aspart through cation chromatography in the present invention is more than 80%.
(2) Due to the Boc-lysine protection at position B₂₉, trypsin digestion does not recognize B₂₉ lysine and does not produce by-products of des(B₃₀), which can improve digestion yield and reduce insulin aspart analog impurities, providing convenience for subsequent purification and separation.
(3) In the process of enzyme digestion, the enzyme digestion yield is improved by optimizing the enzyme addition ratio and controlling the enzyme digestion temperature.
(4) In the step of deprotection, Boc-insulin aspart is converted into insulin aspart, which does not need to be carried out under an organic system, reduces the process steps, environmental pollutions and costs.
(5) The present invention adopts a total of two steps of ion exchange chromatography and one step of reversed-phase chromatography for separation and purification instead of the conventional four-step chromatography, which reduces the production cycle, the use of organic solvents and saves costs.
(6) The fusion protein of the present invention contains a high proportion of the insulin aspart (the fusion ratio increases). The FP or A-FP in the fusion protein contains arginine and lysine and can be digested with proteases into small fragments whose molecular weight is quite different from the target protein, and can readily be separated.

The present invention will be further illustrated below with reference to the specific examples. It should be understood that these examples are only to illustrate the invention, not to limit the scope of the invention. The experimental methods with no specific conditions described in the following examples are generally performed under the conventional conditions, e.g., the conditions described by Sambrook et al., Molecular Cloning: Laboratory Guide (New York: Cold Spring Harbor Laboratory Press, 1989), or according to the manufacturer's instructions. Unless indicated otherwise, all percentage and parts are calculated by weight.

### Example 1 Construction and expression of the insulin aspart expression bacterial strain

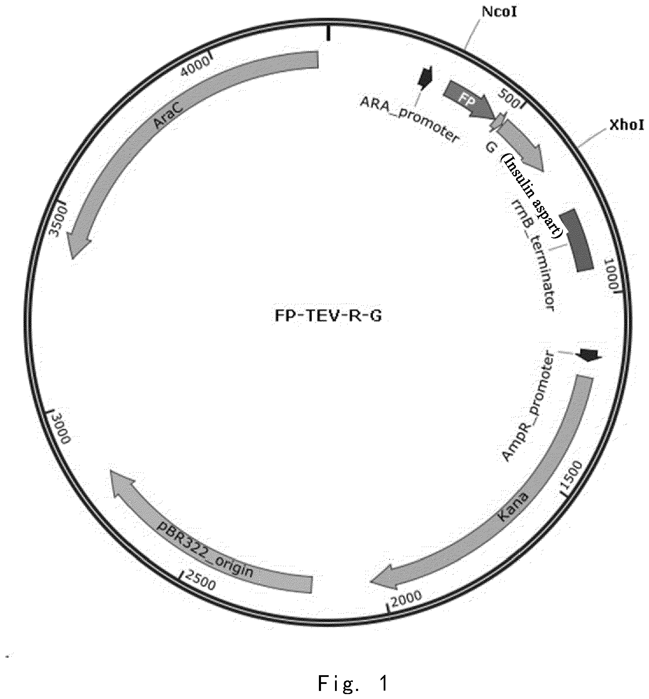

The construction of the insulin aspart expression plasmid refers to the description of Examples in Chinese patent application No. 201910210102.9. The DNA fragment containing the fusion protein FP-TEV-R-G was cloned to the NcoI-XhoI site downstream of the araBAD promoter of the expression vector plasmid pBAD/His A (purchased from NTCC, kanamycin resistance) to obtain the plasmid pBAD- FP-TEV-R-G. The plasmid map is shown in Fig. 1.

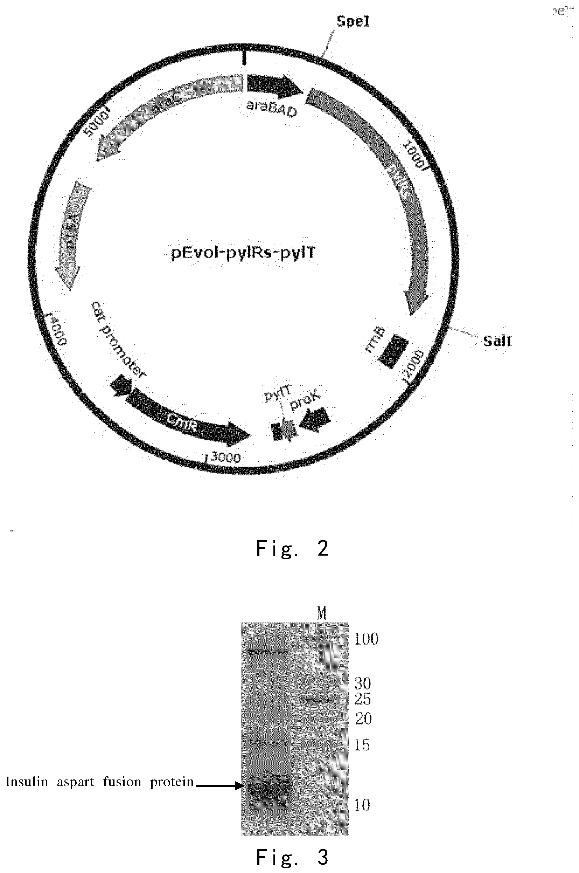

Then the DNA sequence of pylRs was cloned to the SpeI-SalI site downstream of the araBAR promoter of the expression vector plasmid pEvol-pBpF (purchased from NTCC, chloramphenicol resistance), meanwhile the DNA sequence of the tRNA (pylTcua) of lysyl-tRNA synthase was inserted downstream of the proK promoter by PCR. The plasmid is named as pEvol-pylRs-pylT. The plasmid map is shown in Fig. 2.

The constructed plasmids pBAD- FP-TEV-R-G and pEvol-pylRs-pylT were co-transformed into *E. coli* TOP10 strains. The recombinant *E. coli* strains expressing the insulin aspart fusion protein FP-TEV-R-G were screened and obtained. The sequence of FP therein is u8-u9 (SEQ ID NO: 3), and the amino acid sequence of the fusion protein is shown in SEQ ID NO: 1.

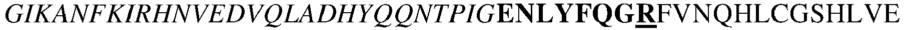

ALYLVCGERGFFYTDK(Boc)TRGIVEQCCTSICSLYQLENYCN (SEQ ID NO:
1)

The following expression plasmids are constructed by using the same method:
pBAD-FP(u9-u10-u11)-TEV-R-G, amino acid sequence of the fusion protein: 
VQLADHYQQNTPIGHYLSTQSVLSKDHMVLLEFVTAAGIENLYFQGRFVNQHL
CGSHLVEALYLVCGERGFFYTDK(Boc)TRGIVEQCCTSICSLYQLENYCN
(SEQ ID NO: 22).
pBAD-FP(u10-u11)-TEV-R-G, amino acid sequence of the fusion protein: 
HYLSTQSVLSKDHMVLLEFVTAAGIENLYFQGRFVNQHLCGSHLVEALYLVCG
ERGFFYTDK(Boc)TRGIVEQCCTSICSLYQLENYCN (SEQ ID NO: 23).
pBAD-FP(gIII)-R-G, amino acid sequence of the fusion protein: M*KKLLFAIPLVVPFYSHSTMELEICSWYHMGIRSFLEQKLISEEDLNSAVD***R**FVNQ HLCGSHLVEALYLVCGERGFFYTDK(Boc)TRGIVEQCCTSICSLYQLENYCN (SEQ ID NO: 10). Among them the FP(gIII) represents the gIII signal peptide derived from green fluorescent protein.

The corresponding expression strains were constructed using conventional methods in the art. The expression of insulin aspart fusion protein was detected by electrophoresis.

| Structure of the fusion protein | Expression of the insulin aspart infusion protein (g/L fermentation broth) |
|---|---|
| FP(u8-u9)-TEV-R-G | 6.5 |
| FP(u9-u10-u11)-TEV-R-G | 6.8 |
| FP(u10-u11)-TEV-R-G | 6.3 |
| FP(gIII)-R-G | 5.3 |

Seed medium was prepared and inoculated, and a secondary seed solution was produced through two-stage culture. After 20 hours of culture, the OD600 reached about 180 and the fermentation was terminated. About 3L of fermentation broth was obtained, and about 130g/L of wet bacteria was obtained by centrifugation. After centrifugation of the fermentation broth, the breaking buffer was added, and bacteria were broken twice by using a high-pressure homogenizer. After centrifugation, Tween 80 and EDTA-2Na were added and then washed once with water, and the inclusion bodies were obtained by centrifugation and collection of the precipitate. About 43g wet weight of inclusion bodies could be finally obtained per liter of fermentation broth.

### Example 2 Dissolution and renaturation of inclusion bodies

8 mol/L urea solution was added to the obtained inclusion bodies, the pH was adjusted to 9.0-10.0 using sodium hydroxide and stirred at room temperature for 1-3 h. The protein concentration was controlled at 10-20 mg/mL. β-mercaptoethanol was added to the final concentration of 15-20mmol/L, and continually stirred for 0.5-1.0 h.

The inclusion body dissolved solution was dripped into the renaturation buffer, diluted to 5-10 times for renaturation. The renaturation solution was maintained at the pH of 9.0-10.0 and stirred for renaturation for 10-20h.

After renaturation for 20 h, the content of insulin aspart fusion protein that was correctly renatured and folded was detected via HPLC. The renaturation rate was over 75%. Fig. 3 shows the SDS-PAGE electropherogram of the insulin aspart fusion protein after renaturation of the inclusion bodies.

### Example 3 Digestion of fusion protein

The renaturation solution was added with dilute hydrochloric acid to adjust the pH to 8.0-9.5, added with recombinant trypsin at the mass ratio of 1:3000 of recombinant trypsin to total protein of the renaturation solution, added with the carboxypeptidase B at the mass ratio of 1:15000 of carboxypeptidase B to total protein of the renaturation solution. The digestion temperature was 36-38 °C and the digestion time was 14-20 h. The Boc-insulin aspart was obtained after digestion.

After digestion for 16h, the content of Boc-insulin aspart in the digestion solution was detected by HPLC. When the difference in the concentration of Boc-insulin aspart detected in two consecutive hours was less than 3%, the digestion was terminated. Finally, the concentration of Boc-insulin aspart in the digestion solution was 0.4-0.6 g/L, and the digestion rate was over 80%.

### Example 4 The first chromatography

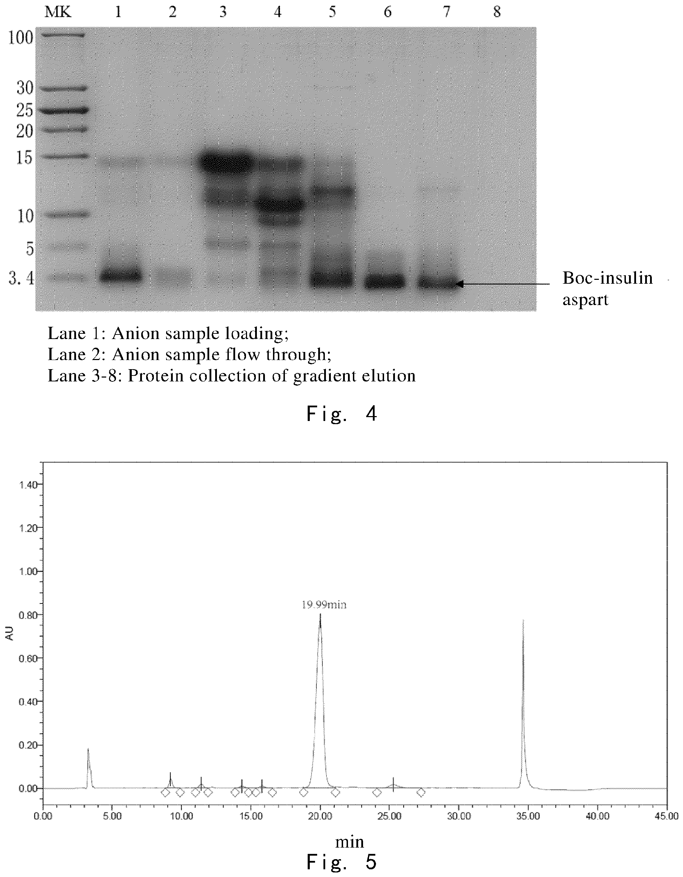

According to the difference of protein isoelectric points, an anion exchange filler was selected for rough extraction of Boc-insulin aspart. The chromatography column was equilibrated with 3-5 column volumes of buffer containing 5-20 mmol/L sodium carbonate at pH 8.0-9.0. Boc-insulin aspart was fully combined with anionic filler and the loading amount was less than 45g/L. After loading, 15 column volumes of 100-500mmol/L sodium chloride was used for linear elution. The eluted protein solution was collected, and the results of SDS protein gel electrophoresis are shown in Fig. 4. The yield of Boc-insulin aspart is over 90%, and the purity is over 70%.

### Example 5 Deprotection

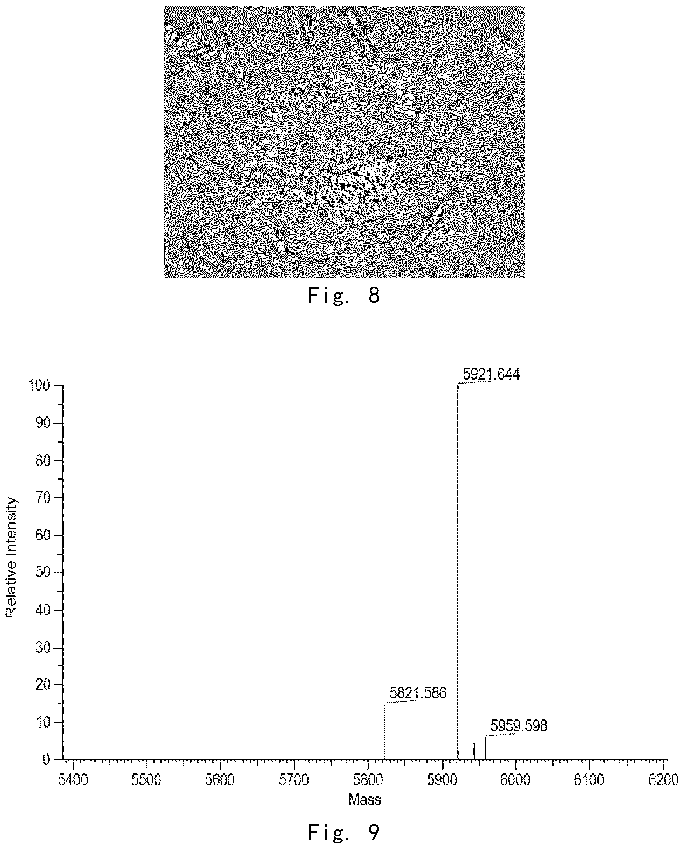

Boc-insulin aspart dry powder was obtained by drying the rough extracted Boc-insulin aspart by anion exchange chromatography. The obtained Boc-insulin aspart dry powder was analyzed by mass spectrometry, which is shown in Fig. 9. The results show that the measured molecular weight of Boc-insulin aspart is 5921.644 Da, and its calculated value is 5925.6 Da, indicating that the protein of interest is obtained.

4-10mL of TFA was added to 1g of Boc-insulin aspart dry powder, stirred and reacted at room temperature 18-25°C for 0.5-3.0h. After the reaction was completed, NaOH was added to adjust the pH of protein solution to over 2.5 to terminate the deprotection reaction. Finally, the insulin aspart was obtained. The deprotection yield detected by HPLC was more than 90% and the purity was more than 75%. The HPLC detection result is shown in Fig. 5.

### Example 6 The second chromatography

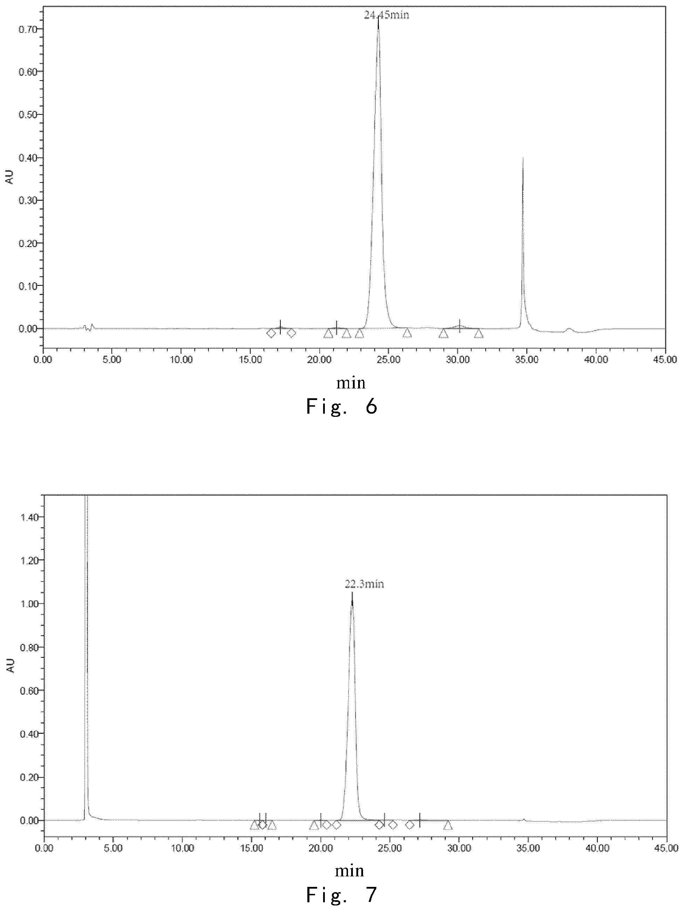

According to the electric charge difference of the substances, insulin aspart was purified via anion exchange chromatography to remove a part of impurities. The ion column was equilibrated with 3-5 column volumes of buffer containing 20 mmol/L glycine at pH 9.0. Boc-insulin aspart protein solution was combined with cation filler and the loading amount was controlled no higher than 10mg/mL. Lastly solution containing 0.27 mol/L of sodium chloride was used for isocratic elution, and the insulin aspart sample was collected. The purity of insulin aspart in the collected solution is 97.83%, and the yield is 87.96%. The HPLC detection result is shown in Fig. 6.

### Example 7 The third chromatography

According to the hydrophobicity difference of the substances, insulin aspart was finely purified by reversed-phase chromatography column technology. The insulin aspart solution obtained by the second chromatography was diluted more than 4 times with pure water and combined with C8 reversed-phase filler. The loading amount of insulin aspart was controlled no higher than 5 mg/mL. 26% acetonitrile containing 200 mmol/L sodium acetate was used for isocratic elution for 10CV. The results show that the yield of insulin aspart is 93.8% and the purity is 99.70%. The detection result is shown in Fig. 7.

### Example 8 Crystallization and lyophilization

Distilled water was added to the collected elution solution of the third chromatography to dilute the acetonitrile solution to a content of no more than 15% (v/v). The solution was added in order with final concentration of 0.7mol/L glycine, 0.8mol/L sodium chloride, 0.5% saturated phenol, then added with zinc acetate at a molar ratio of 1:3, adjusted the pH of the collected elution solution to 5.6-6.0 with acetic acid, and rested for crystallization at 4-8 °C for more than 16h. Rod-like crystal formation was observed under a microscope (see Fig. 8). The crystals were collected and lyophilized to obtain the raw materials for insulin aspart.

All documents mentioned in the present invention are incorporated by reference herein as if each document were incorporated separately by reference. Furthermore, it should be understood that after reading the foregoing teachings of the invention, various changes or modifications may be made to the invention by those skilled in the art and that these equivalents are equally within the scope of the claims appended to this application.

## Claims

1. An insulin aspart fusion protein having the structure as shown in Formula I:
A-FP-TEV-R-G (I)
wherein,
"-" represents a peptide bond;
A is absent or a leader peptide,
FP is a green fluorescent protein folding unit,
TEV is a restriction site, and preferably is a restriction site of TEV enzyme;
R is arginine or lysine used for enzyme digestion;
G is a precursor of insulin aspart or an active fragment thereof;
wherein the green fluorescent protein folding unit comprises 2-6 β-folding units selected from the group consisting of:
| β-folding unit | Amino acid sequence |
|---|---|
| u1 | VPILVELDGDVNG (SEQ ID NO: 11) |
| u2 | HKFSVRGEGEGDAT (SEQ ID NO: 12) |
| u3 | KLTLKFICTT (SEQ ID NO: 13) |
| u4 | YVQERTISFKD (SEQ ID NO: 14) |
| u5 | TYKTRAEVKFEGD (SEQ ID NO: 15) |
| u6 | TLVNRIELKGIDF (SEQ ID NO: 16) |
| u7 | HNVYITADKQ (SEQ ID NO: 17) |
| u8 | GIKANFKIRHNVED (SEQ ID NO: 18) |
| u9 | VQLADHYQQNTPIG (SEQ ID NO: 19) |
| u10 | HYLSTQSVLSKD (SEQ ID NO: 20) |
| u11 | HMVLLEFVTAAGI (SEQ ID NO: 21). |

2. The fusion protein according to claim 1, wherein the green fluorescent protein folding unit is u8-u9, u9-u10-u11, or u10-u11.

3. The fusion protein according to claim 1, wherein the G is a Boc (tert-butoxycarbonyl)-modified insulin aspart precursor which has the structure as shown in Formula II:
GB- X-GA (II)
wherein,
GB is the B chain of insulin aspart modified with Boc at position 29, whose amino acid sequence is shown in positions 1-30 of SEQ ID NO: 5,
X is a linker peptide;
GA is the A chain of insulin aspart whose amino acid sequence is shown in positions 32-52 of SEQ ID NO: 5.

4. The fusion protein according to claim 1, wherein the sequence of the insulin aspart fusion protein is shown in SEQ ID NO. 1, 22, 23.

5. A double chain insulin aspart fusion protein having the structure as shown in Formula III:
A-FP-TEV-R-D (III)
wherein,
"-" represents a peptide bond;
A is absent or a leader peptide,
FP is a green fluorescent protein folding unit,
TEV is a restriction site, and preferably is a restriction site of TEV enzyme;
R is arginine or lysine used for enzyme digestion;
D is a Boc-modified double chain insulin aspart having the structure as shown in the following Formula IV; 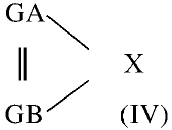
wherein, " 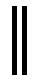 " represents a disulfide bond;
GA is the A chain of insulin aspart whose amino acid sequence is shown in positions 32-52 of SEQ ID NO: 5,
X is a linker peptide;
GB is the B chain of insulin aspart modified with Boc at position 29, whose amino acid sequence is shown in positions 1-30 of SEQ ID NO: 5;
wherein the green fluorescent protein folding unit comprises 2-6 β-folding units selected from the group consisting of:
| β-folding unit | Amino acid sequence |
|---|---|
| u1 | VPILVELDGDVNG (SEQ ID NO: 11) |
| u2 | HKFSVRGEGEGDAT (SEQ ID NO: 12) |
| u3 | KLTLKFICTT (SEQ ID NO: 13) |
| u4 | YVQERTISFKD (SEQ ID NO: 14) |
| u5 | TYKTRAEVKFEGD (SEQ ID NO: 15) |
| u6 | TLVNRIELKGIDF (SEQ ID NO: 16) |
| u7 | HNVYITADKQ (SEQ ID NO: 17) |
| u8 | GIKANFKIRHNVED (SEQ ID NO: 18) |
| u9 | VQLADHYQQNTPIG (SEQ ID NO: 19) |
| u10 | HYLSTQSVLSKD (SEQ ID NO: 20) |
| u11 | HMVLLEFVTAAGI (SEQ ID NO: 21). |

6. A Boc-modified insulin aspart precursor having the structure as shown in Formula II:
GB- X-GA (II)
wherein,
GB is the B chain of insulin aspart modified with Boc at position 29, whose amino acid sequence is shown in positions 1-30 of SEQ ID NO: 5,
X is a linker peptide, and the amino acid sequence of X is R, RR, RRR or as shown in SEQ ID NO: 6-9; and
GA is the A chain of insulin aspart whose amino acid sequence is shown in positions 32-52 of SEQ ID NO: 5.

7. A Boc-modified double chain insulin aspart having the structure as shown in Formula IV: 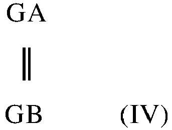 wherein, " 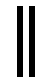 " represents a disulfide bond;
GA is the A chain of insulin aspart whose amino acid sequence is shown in positions 32-52 of SEQ ID NO: 5,
GB is the B chain of insulin aspart whose amino acid sequence is shown in positions 1-30 of SEQ ID NO: 5, and the lysine at position 29 of the B chain is Nε-(tert-butoxycarbonyl)-lysine.

8. An isolated polynucleotide encoding the insulin aspart fusion protein of claim 1, the double chain insulin aspart fusion protein of claim 5, the Boc-modified insulin aspart precursor of claim 6, or the Boc-modified double chain insulin aspart of claim 7.

9. A vector comprising the polynucleotide of claim 8.

10. A host cell comprising the vector of claim 9, or in which the chromosome is integrated with exogenous polynucleotide of claim 8, or expressing the insulin aspart fusion protein of claim 1, the double chain insulin aspart fusion protein of claim 5, the Boc-modified insulin aspart precursor of claim 6, or the Boc-modified double chain insulin aspart of claim 7.

11. A method for preparing insulin aspart, comprising the steps:
(i) using recombinant bacteria to ferment to prepare a fermentation broth containing the insulin aspart fusion protein of claim 1 (primary protein);
(ii) digesting the insulin aspart fusion protein (primary protein) with enzyme, thereby obtaining a Mixture I containing the Boc-modified insulin aspart (secondary protein);
(iii) deprotecting the Boc-modified insulin aspart (secondary protein), thereby obtaining a Mixture II containing the deprotected insulin aspart (tertiary protein);
(iv) purifying the Mixture II, thereby obtaining the insulin aspart.

12. The method according to claim 11, wherein between step (ii) and step (iii) it further comprises the step:
(I) performing the first anion exchange chromatography on the Mixture I, thereby obtaining dry powder containing the Boc-modified insulin aspart (secondary protein).

13. The method according to claim 11, wherein the purification in step (iv) further comprises the steps:
(II) performing the second anion exchange chromatography, which uses glycine as the mobile phase on the Mixture II, thereby obtaining an Eluate II containing the deprotected insulin aspart (tertiary protein).
(III) performing reversed-phase chromatography on the Eluate II, thereby obtaining the insulin aspart.

14. The method according to claim 11, wherein in step (i), insulin aspart fusion protein inclusion bodies are isolated from the fermentation broth of the recombinant bacteria, and the inclusion bodies are denatured and renatured to obtain the insulin aspart fusion protein (primary protein) with correct protein folding.

15. The method according to claim 11, wherein in step (ii), trypsin and carboxypeptidase B are used for the digestion.
